# EUROPEAN PATENT APPLICATION

(11) **EP 2 385 105 A2**
(43) Date of publication of application: **09.11.2011**
(21) Application number: 11002580.6
(22) Date of filing: 01.09.2009
(51) Int. Cl.: C12N 5/00, A61L 27/48, A61L 27/52, A61L 27/58, A61P 19/00

(54) **Polymer blends**

(30) Priority: 01.09.2008 GB 0815883
(62) Divisional of application: 09785038.2
(71) Applicant: The University Court Of The University of Edinburgh, South Bridge Edinburgh EH8 9YL (GB)
(72) Inventor: Oreffo, Richard O.C., Southampton SO16 6YD (GB); Tare, Rahul Shrikan, Southampton SO16 6YD (GB); Bradley, Mark, Edinburgh EH9 3JJ (GB); Khan, Ferdous, Edinburgh EH9 3JJ (GB)
(74) Representative: Chapman, Paul Gilmour

(57) **Abstract**

A biocompatible polymer mixture for use as a matrix for cellular attachment includes a mixture of at least two polymers selected from the group consisting of:
chitosan (CS), polyethylenimine (PEI), poly(L-lactic acid) (PLLA), poly(D-lactic acid)(PDLA), poly(2-hydroxyethyl methacrylate) (PHEMA), poly(ε-caprolactone) (PCL), poly(vinyl acetate) (PVAc), poly (ethylene oxide) (PEO), poly[(R)-3-hydroxybutyric acid)] (PHB), cellulose acetate (CA), poly(lactide-co-glycolide) (PLGA) and poly(N-isopropylacrylamide)(PNIPAM). Implants making use of the polymer mixtures can support cell attachment, growth and differentiation, and tissue regeneration *in vivo.*

## Description

### Field of the invention

The present invention relates to the provision of improved polymers, especially mixtures of polymers that can find use as biocompatible materials, which support cell attachment, growth and differentiation, and tissue regeneration in vivo.

### Background of the Invention

Biomaterials have been defined as substrates other than foods or drugs of natural or synthetic origins, that comprises whole or part of a living structure or biomedical device which performs, augments, or replaces a natural function. The use of polymers as biomaterials is having an enormous impact in the world of medical science¹⁻³. For example the use of polymer-coated stents, is a potential technique to achieve high local tissue concentrations of an effective drug at the precise site during vessel injury.⁴ Thiolated poly(acrylic acid) coated stents sustain release of a model peptide drug⁵. Combining polymers with mammalian cells, it is now possible to regenerate skin for patients who have burns or skin ulcers; various other polymer/cell combinations are in clinical trials ⁷. The search for ideal biomaterials is typically an iterative process for tissue regeneration, where the properties of the scaffolds employed to support cells are dictated concurrently by a number of factors. These include the kinetics of cell growth, scaffold degradation and the mechanical properties required at the site of implant, as well as cell-material interactions⁸.

Current methods for the development of biomaterials require the preparation and characterization of individual materials; this is neither efficient nor cost effective. Alternatively the application of a high-throughput approach to material design and discovery ¹⁶⁻¹⁸ can be used to accelerate both the discovery and selection of suitable materials. An approach that shows promise in creating new biomaterials is that of polymer blends ¹³⁻¹⁵ . An attractive feature of blending (mixing) is the ease of combining several materials of a vast variety of characteristics into architectures with properties that are often not accessible with individual components. For instance, the cell attachment and proliferation on the surface of polymeric materials can be improved with respect to those of single materials by blending them with judiciously selected other polymers⁸. For functional performance, the design of a material is important, for example materials (polymers) having two separate phases being used to create devices for specific medical applications ¹⁹, which are known as shape-memory material.

It is well recognized that human bone marrow mesenchymal stem cells (hBMSCs) can give rise to cells of the stromal lineage, such as adipogenic (fat), chondrogenic (cartilage), osteoblastic (bone), myoblastic (muscle) and fibroblastic (connective tissue) cells and are able to generate intermediate progenitors with a degree of plasticity. Thus, hBMSCs can give rise to a hierarchy of bone cell populations with a range of developmental stages, including the MSC, determined osteoprogenitor cell, preosteoblast, osteoblast and, ultimately, the osteocyte¹⁹⁻²². An ideal orthopaedic repair material would thus influence this heterogeneous progenitor cell mix in *vivo* to produce mature osteoblasts, rather than connective tissue cell types. The generation of mature osteoblast populations would allow the production of osteoid including collagen type I and calcium phosphate (apatite) mineral, which is essential for new bone formation.

It is an object of the invention to provide polymer blends with improved properties with respect to supporting at least one of cell attachment growth and differentiation. It is an object of the present invention to provide implants for use in the reconstruction of tissue, especially orthopedic tissue for the formation or repair of bone, spine, etc. It is an object of the present invention to provide methods for obtaining improved polymer blends and for testing their application as scaffolds for cell attachment and growth.

### Summary of the Invention

According to a first aspect, the present invention provides a biocompatible polymer mixture for use as a matrix for cellular attachment comprising a mixture of at least two polymers, selected from the group consisting of: chitosan (CS), polyethylenimine (PEI), poly(L-lactic acid) (PLLA), poly(D-lactic acid)(PDLA), poly(2-hydroxyethyl methacrylate) (PHEMA), poly(s-caprolactone) (PCL), poly(vinyl acetate) (PVAc), poly(ethylene oxide) (PEO), poly[(R)-3-hydroxybutyric acid] (PHB), cellulose acetate (CA), poly(lactide-co-glycolide) (PLGA) and poly(N-isopropylacrylamide)(PNIPAM).

These polymers have been found to be effective in binary (two component) and ternary (three component) mixtures, which can provide good cell attachment, growth and differentiation. Blends comprising more than three polymer components may also be made.

The structures of these polymers are indicated in Scheme 1 below.

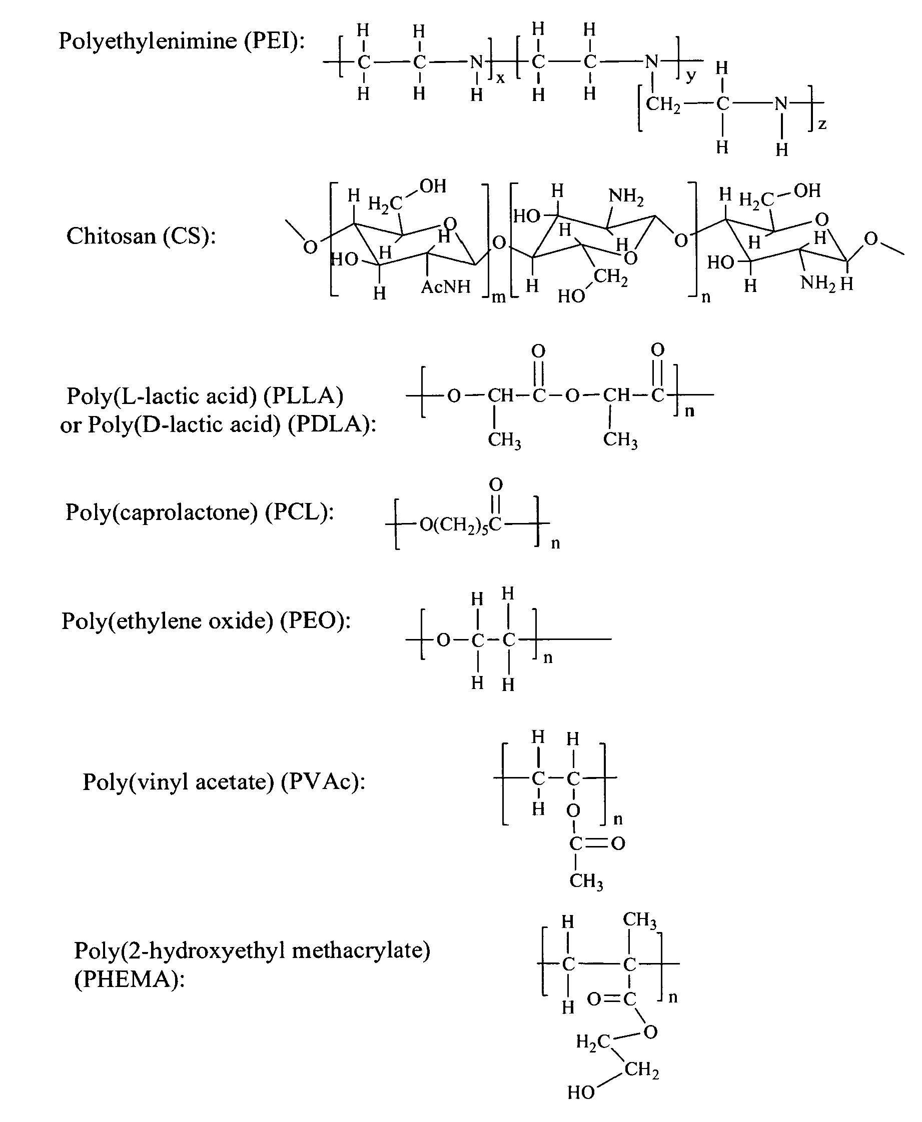

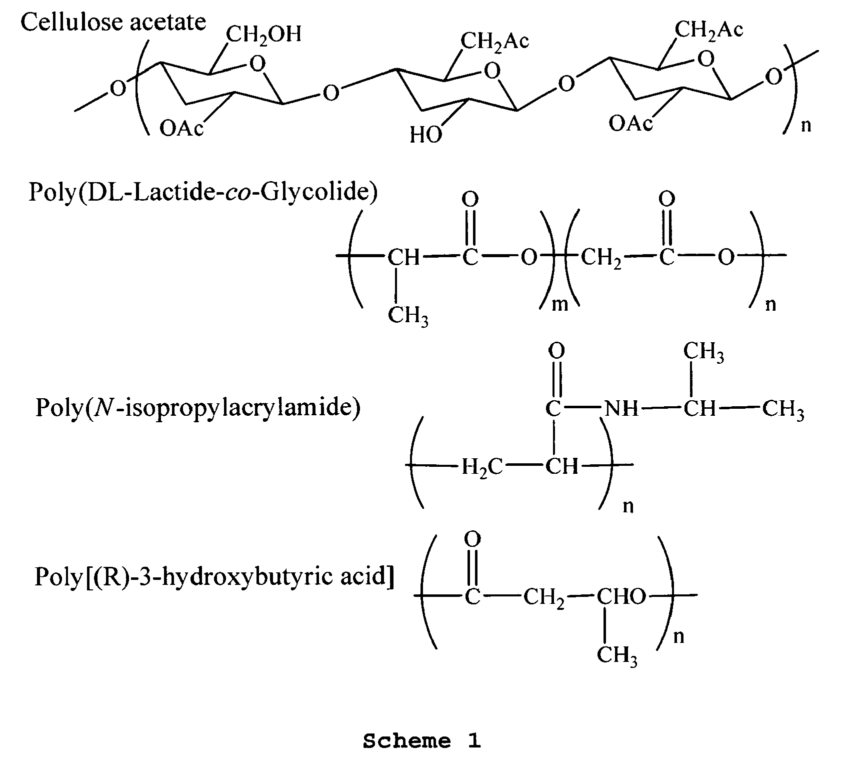

In addition to allowing cellular attachment the biocompatible polymer mixture may support cell growth. Preferably the biocompatible polymer mixture supports cell differentiation i.e. the matrix allows cells such as stem cells to grow and differentiate in a manner akin to that found in nature. Thus the biocompatible polymer mixtures of the invention may be employed to support the growth of both soft (for example muscle, skin and nerves) and hard (for example bone, spine, cartilage) tissues.

Preferably the biocompatible polymer mixture provides a matrix to promote bone formation, repair spine and cartilage.

Surprisingly, it has been found that it is possible to prepare polymer mixtures (blends), which have structures suitable for use as scaffolding for the formation of tissue, for example bone, by making an appropriate mixture of polymers. It is not necessary to react (co-polymerise) the polymer components together to obtain a new material with useful structural properties. The intimate mixtures of the two or more polymer components provided by the present invention can provide scaffold structures that are both biocompatible and biodegradeable.

The polymer mixtures may be prepared by dissolving the selected polymer components in a suitable solvent and then removing the solvent from the mixture e.g. by heating to evaporate the solvent and thus provide the polymer mixture with the desired composition. Conveniently the polymer components are dissolved separately in suitable solvents and the resulting solutions mixed in a selected ratio to provide a mixed polymer solution that is then processed to remove the solvent. The ratios described herein are in terms of weight: weight of each component. Alternative methods of production may be employed, for example dissolving all the polymer components in a suitable solvent and then processing to a dried product.

The polymer mixture is biocompatible. It will be understood that the term biocompatible means that the polymer mixture is at least not toxic to cells or living tissue.

Surprisingly it has been found possible to produce biocompatible polymer mixtures that include relatively high amounts of a polymer that is, on it's own, toxic to some cell lines. For example a polymer mixture containing significant amounts of polyethylenimine (PEI), which is toxic to certain cells. PEI has the further disadvantage that, on its own, it is soluble in aqueous systems, which makes it generally unsuitable for use as an implant, except when made into a polymer mixture of the invention.

Advantageously the polymer mixture provides a scaffold that promotes superior cell growth and differentiation in comparison to growth and differentiation in similar conditions but in the absence of the polymer.

Suitable binary (two component) polymer mixtures identified as providing at least good cell attachment in some embodiments of the invention include: polyethylenimine/ poly(vinyl acetate)(PEI/PVAc), polyethylenimine/poly(2-hydroxyethyl methacrylate) (PEI/PHEMA),
polyethylenimine/poly(ethylene oxide)
(PEI/PEO),polyethylenimine /chitosan (PEI/CS), polyethyleneimine/ poly(ε-caprolactone)(PEI/PCL), poly(1-lactic acid)/poly(ε-caprolactone) (PLLA/PCL), poly(D-lactic acid)/chitosan (PDLA/CS), poly(D-lactic
acid)/polyethylenimine (PDLA/PEI) poly[(R)-3-hydroxybutyric acid)]/chitosan (PHB/CS), poly[(R)-3-hydroxybutyric acid)] / polyethylenimine (PHB/PEI), cellulose acetate / polyethylenimine (CA/PEI), cellulose acetate/chitosan (CA/CS), poly(Lactide-co-glycolide)/ chitosan (PLGA/CS), poly(Lactide-co-glycolide)/polyethylenimine (PLGA/PEI), poly(N-isopropylacrylamide)/chitosan(PNIPAM/CS), poly(N-isopropylacrylamide)/ polyethylenimine (PNIPAM/PEI), poly(N-isopropylacrylamide)/ poly(ε-caprolactone) ( PNIPAM/PCL) , poly(N-isopropylacrylamide)/ poly(L-lactic acid)(PNIPAM/PLLA), poly(N-isopropylacrylamide)/ poly(D-lactic acid)(PNIPAM/PDLA), poly(N-isopropylacrylamide) / poly[(R)-3-hydroxybutyric acid)](PNIPAM/PHB), poly(N-isopropylacrylamide)/cellulose acetate(PNIPAM/CA), poly(N-isopropylacrylamide)/ poly(2-hydroxyethyl
methacrylate)(PNIPAM/PHEMA), and poly(N-isopropylacrylamide)/ poly(vinyl acetate)(PNIPAM/PVAc).

In other embodiments of the invention a suitable binary (two polymer component) mixture of polymers may be one of: polyethylenimine/ poly(vinyl acetate)(PEI/PVAc), polyethylenimine/poly(2-hydroxyethyl methacrylate)
(PEI/PHEMA), polyethylenimine/poly(ethylene oxide)
(PEI/PEO),polyethylenimine /chitosan (PEI/CS), polyethyleneimine/ poly(ε-caprolactone) (PEI/PCL) and poly(L-lactic acid) / poly (ε-caprolactone) (PLLA/PCL).

Typically the ratio of the two components of the binary mixtures may be from 5:95 to 95:5.

For example, mixtures containing polyethylenimine (PEI) having a ratio of the two components in the range from 10:90 to 90:10 of PEI to the second component have been shown to exhibit good cell attachment, in contrast to PEI alone, which did not show good cell attachment and is toxic to certain cells.

For example, in tests good attachment of human bone marrow-derived STRO-1+ progenitor cells has been found for PEI/PCL mixtures with ratios of the two components varying from 40:60 to 90:10. Comparable results were obtained for PLLA/PCL mixtures with ratios of 10:90 to 60:40. PEI/CS mixtures also gave good attachment results with STRO-1+ cells.

Ternary mixtures that exhibit good cell attachment include:
PEI/PCL/PLLA, PEI/PEO/PLLA, PEI/PVAc/PEO, PEI/pHEMA/PCL, PLLA/PEI/pHEMA, PLLA/PEO/PCL, PLLA/PVAc/pHEMA, PLLA/pHEMA/CS, PLLA/PCL/CS, CS/PEI/PLLA, CS/PEO/PEI, CS/PVAc/PEI, CS/PVAc/PEO, CS/PVAc/PCL, CS/pHEMA/PEI, CS/PCL/PEI and CS/PLLA/PEO.

Typical ternary mixtures may be prepared with up to 90 parts of one component and five parts of each of the other two (5:5:90). Most of these mixtures support both cell attachment and growth, for example, CS/PVAc/PEI, PEI/pHEMA/PCL and PEI/PVAc/PEO.

Binary polymer mixtures of poly(L-lactic acid)/poly(ε-caprolactone) (PLLA/PCL) and mixtures of polyethylenimine/chitosan (PEI/CS) have been found to have a particularly useful structure. These two types of mixture have been found to exhibit micro porous structures that can resemble, on microscopic inspection, the micro porous structure of bone.

This apparent similarity to a natural bone structure is continued when these mixtures are tested with regard to their ability to attach cells and allow them to grow and differentiate. For example fetal stem cells may be successfully attached to a polymer mixture and if the composition is placed in a suitable growth environment then the cells will grow for periods of at least four weeks, as described later with reference to specific embodiments.

A mixture of PLLA/PCL (for example in the ratio 20:80) was found to exhibit a particularly good porous structure, comparable to natural bone porosity and has been shown to aid bone healing in femur defects of mouse test subjects.

The results demonstrate that polymer mixtures of the invention, for example a PLLA/PCL (20:80) mixture can support the generation of new bone/osteoid to restore the lost bone tissue in the defect region. Bone regeneration was observed in subjects fitted with a PLLA/PCL (20:80) scaffold alone after 28 days. Where the PLLA/PCL (20:80) scaffold was seeded with human skeletal stem cells, the results were significantly improved. (The indices of bone histomorphometry were significantly enhanced).

Although PCL and PLLA are FDA approved materials for clinical use, PCL is known to have a slow degradation rate (several years depending on the processing conditions), while PLLA is known to have relatively fast degradation rate. The example PLLA/PCL (20:80) scaffold integrates the properties of both polymers. Modifying the ratio of components and/or adding a further component or components to the mixture, for example, can thus be employed to tune the degradation rate or the prosity.

Further modifications may be made by seeding the polymer mixture scaffold with other components, such as growth factors. For example bone morphogenetic proteins (BMPs), Epidermal growth factor (EGF), Fibroblast growth factor (FGF), Erythropoietin (EPO), Granulocyte-colony stimulating factor (G-CSF) , Granulocyte-macrophage colony stimulating factor (GM-CSF) , Growth differentiation factor-9 (GDF9), Hepatocyte growth factor (HGF) , Insulin-like growth factor (IGF), Myostatin (GDF-8), Nerve growth factor (NGF) and other neurotrophins, Platelet-derived growth factor (PDGF), Thrombopoietin (TPO), Transforming growth factor- β1 (TGF-β1) and Vascular endothelial growth factor (VEGF).

Mixtures were made from polyethylenimine (PEI) and chitosan (CS), formed from an aqueous system, and resulted in the formation of hydrogels. The combination of these two components from an aqueous system resulted in a mixture that incorporated substantial quantities of water producing a gel structure.

These biocompatible hydrogels comprising polyethylenimine (PEI) chitosan (CS) and water are of particular interest and constitute a second aspect of the invention. Other polymer components may be included to form three or more component mixtures. Preferred binary mixtures (containing only chitosan and polyethylenimine as polymer components) contain chitosan/PEI in ratios of from 10:90 to 90:10. A most preferred blend has a chitosan/PEI ratio of 40:60.

The hydrogels may be dried (substantially freed from water) for storage prior to use, if desired. The drying process employed may be freeze drying. Freeze drying has the advantage of preserving the three dimensional porous or fibrous structure of the polymer part (polymer mixture) of the hydrogel.

Hydrogels have attracted considerable attention as so-called "smart biomaterials" due to the various and often intriguing physical and chemical phenomena they can display when subjected to a variety of external stimuli, such as changes in pH, solvent, ionic strength, temperature, light and electric field.¹⁻² As a result, hydrogels have also been applied as fundamental components in a variety of medical devices such as tissue engineering scaffolds³ and controlled drug-delivery systems⁴ and as soft linear actuators, sensors, and energy transducing devices¹⁻², ⁵ in a range of novel applications.

Known hydrogel synthesis methods include the cross-linking of linear poly (N-isopropylacrylamide) (PNIPAAm)⁵, poly(ethylene glycol)⁶, poly (acrylamide)⁷, poly(acrylic acid)-based polymers⁸ and their copolymers^{9,10}.

These types of hydrogel follow either a positive thermoreversible system (polymer solutions having an upper critical solution temperature (UCST) which shrink when cooled below UCST) or negative thermoreversible system (polymer solutions having a lower critical solution temperature (LCST) which contract by heating above the LCST). However, the use of previously known polymers in cell therapeutic applications is limited due to their toxicity and non-biodegradability. ⁵

Chitosan on its own has been used to form so called "hydrogels" via the neutralization of acidic solutions of chitosan. However, the resulting materials are opaque, with a granular morphology which lacks real physical stability and are more akin to a precipitate than a real "hydrogel". They also show a crystalline morphology.¹¹ For use as a biomaterial chitosan and PEI have also been chemically grafted together to give materials with enhanced gene carrier abilities.¹²

Surprisingly, biologically compatible hydrogels, with a stable morphology and the ability to support cell growth were readily prepared by the simple expedient of mixing of aqueous chitosan (CS) and polyethylenimine (PEI) solutions, as described hereafter with reference to specific examples. The gels may be dried prior to use and rehydrated when seeded with cells, for example.

The gels were found to have good thermal stability, for example a chitosan/PEI (40/60) hydrogel showed little change in volume at temperatures between 22 and 40°C. Substantial shrinkage did occur on heating this gel to higher temperatures (up to 90°C) but there was no tendency for the gel to collapse or fragment. Importantly for use in biological applications the gels were found to be stable at physiological pH (7.4) for several weeks.

The hydrogel mixtures may be formed by mixing appropriate aqueous solutions of chitosan and polyethylenimine. Advantageously for some applications the hydrogels may be formed in a cell culture medium. By this means the hydrogels may be readily seeded with cells which can be incubated in the culture medium, growing and supported by the matrix or scaffold provided by the polymer mixture's structure.

Hydrogels have been used as three dimensional (3D) scaffolds in tissue engineering applications to mimic the natural extra cellular matrix (ECM) and to support cellular adhesion, migration and proliferation. The novel CS/PEI hydrogels supported the attachment and growth of mammalian cells as shown by using a microarray of the present invention as described below. The hydrogels were examined alongside samples consisting of chitosan and PEI alone. The homopolymers (chitosan and PEI) alone did not support cell attachments or spreading, while chitosan/PEI mixture showed significant cell attachment.

A preferred mixture (chitosan/PEI: 40/60) supported cell attachment and spreading. In experiments, labelled cells were seeded in the chitosan/PEI gel by mixing with the appropriate chitosan /PEI mixture, before full gelation had occurred. Adding cells to the hydrogel in this manner homogeneously distributed the cells throughout the resulting scaffold. The cells were found to be viable in the gels when the culture was continued for up to four weeks.

Advantageously, the polymer mixtures and hydrogel polymer mixtures of the invention are biodegradable i.e. the polymer mixture when placed in a subject will degrade over a period of time so that polymer is eliminated from the subjects body. A biodegradable polymer mixture has the advantage that when a portion of polymer mixture of the invention is placed within a subject to replace damaged tissue, for example bone, cartilage and nerve, the polymer mixture structure will be replaced by growing cells, conforming to the scaffold provided by the portion of polymer mixture.

Polymer mixtures of the invention that are biodegradable include but are not limited to CS/PEI, PLA/PCL, CS/PLLA, CS/PCL, PEI/PCL and PEI/PLLA. By biodegrading after a period sufficient to allow the growth of living tissue, the portion of polymer mixture can act as a temporary structure, which will be replaced in time by grown tissue. The biodegradable polymer mixture thus affords a method for repair or replacement of damaged tissue by natural or modified (for example genetically engineered) natural tissue, appropriate to the part of the body being repaired or replaced, rather than by replacement with a manufactured substitute.

According to a third aspect, the present invention provides an implant for the repair or replacement of living tissue comprising a biocompatible polymer mixture according to the first or second aspects of the invention.

The implant may include living cells attached to the biocompatible polymer mixture. For example adult human bone marrow-derived skeletal stem/ progenitor cells, human fetal skeletal progenitor cells or human articular chondrocytes.

Alternatively the implant may be incubated with suitable cells, in vitro, prior to use, to provide an implant comprising tissue, which may be natural tissue or modified or genetically engineered natural tissue.

Alternatively the implant may be used without attached cells or tissue whereupon it may be colonised by the subject's own cells, providing a matrix or scaffold for growth of the cells.

Tissues that may be repaired or replaced by the implant of the invention include bone or cartilage. Other tissues, for example soft tissues such as muscle, skin or nerve may also be repaired or replaced.

The implant may simply consist of a polymer mixture of the invention with or without attached cells. Other components may be included in the implant. For example, the implant may include DNA, RNA, proteins, peptides or therapeutic agents for treatment of disease conditions. The implant may also include biodegradable and non-biodegradable components.

For example, the implant may be a stent of a manufactured non-biodegradable material but coated with a selected polymer mixture of the invention and optionally seeded with appropriate cells.

For further example, the implant may be used for replacement of bone and may include a permanent support such as a steel plate or pin and a portion made from a polymer mixture of the invention and seeded with bone producing cells. In use the steel plate or pin remains as a structural support, whilst the polymer mixture acts as a scaffold but degrades following the desired growth of bone tissue.

The implants of the invention may be used to effect tissue repair or replacement. Thus according to a fourth aspect the present invention, provides a method for repair or replacement of tissue comprising: providing an implant according to the third aspect of the invention; and locating the implant on or in the body of a subject.

For example, the implant may be placed on the body of a subject when skin tissue is being repaired. For further example, the implant may be placed within a subject when bone or an internal organ is being repaired.

Selecting suitable biocompatible polymer mixtures for different applications can be onerous, requiring the preparation of a large number of samples and careful testing to ensure that the polymer mixture complies with all the relevant criteria for successful use such as good attachment and growth of cells. Accordingly high throughput methods have been adapted for use in testing large numbers of polymer mixture samples. A microarray of polymer mixture samples has been found to be a highly convenient tool for testing and comparing samples of the polymer mixtures of the invention. The microarrays constitute a fifth aspect of the present invention.

The microarray of polymer blends may be prepared by the following method. A plurality of polymer solutions is prepared. Each solution contains a mixture of at least two polymers prepared in selected concentrations. The solutions are then printed as an array of microspots onto a substrate and the microspots are dried to form a microarray of dried polymer spots attached to the substrate.

The substrate is preferably agarose or another substrate that cells do not tend to adhere to. Use of a substrate which cells do not adhere to has the advantage of avoiding false positives on testing caused by cells adhering to the substrate rather than to the polymer blend under test. Agarose supported on a glass plate has been found to be a satisfactory base for printing the microarray.

The microarray can be used of testing the ability of a large number of polymer mixtures to support cell attachment, growth and/or differentiation.

After growing the cells on the microarray an assessment of the amount of cell attachment and growth may be made. The assessments of the suitability of a given polymer mixture of the attachment and /or the growth of a particular cell type may be carried out in a number of ways. The microarray may be examined visually by microscope. Alternatively the assessment may be automated. For example the microarray may be examined by a vision system, which assesses the growth of cells by comparing images of each microspot before and after the incubation step.

The cells may be marked, for example with a fluorescent marker by methods well known to those skilled in the art. For example on illumination with appropriate light the microspots of polymer mixture will exhibit fluorescence that is dependent on the quantity of cells attached to and growing on a given microspot. The measurement of the amount of fluorescence can be automated by fluorescence microscopy combined with analysis using an appropriate software program in the usual fashion.

Polymer mixtures exhibiting good cellular attachment and potential for cell growth such as those described above have been found by making use of these microarrays in a testing programme.

According to a sixth aspect the present invention provides a biocompatible polymer mixture for use as a matrix for cellular attachment comprising a mixture of at least two polymers selected from the group consisting of:
chitosan (CS), polyethylenimine (PEI), poly(L-lactic acid) (PLLA), poly(D-lactic acid)(PDLA), poly(2-hydroxyethyl methacrylate) (PHEMA), poly(ε-caprolactone) (PCL), poly(vinyl acetate) (PVAc), poly (ethylene oxide) (PEO), poly[(R)-3-hydroxybutyric acid)] (PHB), cellulose acetate (CA), poly(lactide-co-glycolide) (PLGA) and poly(N-isopropylacrylamide)(PNIPAM).

The biocompatible polymer mixture may comprise two polymer components, selected from the group consisting of polyethylenimine/ poly(vinyl acetate)(PEI/PVAc), polyethylenimine/poly(2-hydroxyethyl methacrylate)
(PEI/PHEMA), polyethylenimine/poly(ethylene oxide) (PEI/PEO),polyethylenimine /chitosan (PEI/CS), polyethyleneimine/ poly(ε-caprolactone)(PEI/PCL), poly(L-lactic acid)/poly(ε-caprolactone) (PLLA/PCL), poly(D-Lactic acid)/chitosan (PDLA/CS), poly(D-Lactic
acid)/polyethylenimine (PDLA/PEI) poly[(R)-3-hydroxybutyric acid)]/chitosan (PHB/CS), poly[(R)-3-hydroxybutyric acid)] / polyethylenimine (PHB/PEI), cellulose acetate / polyethylenimine (CA/PEI), cellulose acetate/chitosan (CA/CS), poly(Lactide-co-glycolide)/ chitosan (PLGA/CS), poly(Lactide-co-glycolide)/polyethylenimine (PLGA/PEI), poly(N-isopropylacrylamide)/chitosan(PNIPAM/CS), poly(N-isopropylacrylamide)/ polyethylenimine (PNIPAM/PEI), poly(N-isopropylacrylamide)/ poly(ε-caprolactone)
(PNIPAM/PCL), poly(N-isopropylacrylamide)/ poly(L-lactic acid)(PNIPAM/PLLA), poly(N-isopropylacrylamide)/ poly(D-Lactic acid)(PNIPAM/PDLA), poly(N-isopropylacrylamide) / poly[(R)-3-hydroxybutyric acid)](PNIPAM/PHB), poly(N-isopropylacrylamide)/cellulose acetate(PNIPAM/CA), poly(N-isopropylacrylamide)/ poly(2-hydroxyethyl
methacrylate)(PNIPAM/PHEMA), and poly(N-isopropylacrylamide)/ poly(vinyl acetate)(PNIPAM/PVAc).

The two polymer components may be selected from the group consisting of: polyethylenimine/ poly(vinyl acetate)(PEI/PVAc), polyethylenimine/poly(2-hydroxyethyl methacrylate) (PEI/PHEMA), polyethylenimine/poly(ethylene oxide) (PEI/PEO),polyethylenimine /chitosan (PEI/CS), polyethyleneimine/ poly(ε-caprolactone)(PEI/PCL), poly(L-lactic acid)/poly(ε-caprolactone) (PLLA/PCL).

The biocompatible polymer mixture comprising two polymer components may have a microporous structure and comprise two polymer components selected from the group consisting of poly(L-lactic acid)/poly(ε-caprolactone) (PLLA/PCL) and polyethylenimine/chitosan (PEI/CS).

Such a biocompatible polymer mixture may be one where the polymer components of the mixture are poly(L-lactic acid)/poly(ε-caprolactone) (PLLA/PCL) having a ratio in the range from 10:90 to 60:40.

The poly(L-lactic acid)/poly(ε-caprolactone) (PLLA/PCL) may be in the ratio of 20:80.

In the biocompatible polymer mixture according to the sixth aspect the mixture may be a two component mixture containing polyethylenimine (PEI) and having a ratio in the range from 10:90 to 90:10 of PEI to the second component.

In the biocompatible polymer mixture according the sixth aspect the mixture may comprise three polymer components selected from the group consisting of PEI/PCL/PLLA, PEI/PEO/PLLA, PEI/PVAc/PEO, PEI/pHEMA/PCL, PLLA/PEI/pHEMA, PLLA/PEO/PCL, PLLA/PVAc/pHEMA, PLLA/pHEMA/CS, PLLA/PCL/CS, CS/PEI/PLLA, CS/PEO/PEI, CS/PVAc/PEI, CS/PVAc/PEO, CS/PVAc/PCL, CS/pHEMA/PEI, CS/PCL/PEI and CS/PLLA/PEO.

In the biocompatible polymer mixture according to the sixth aspect the mixture may comprise polyethylenimine (PEI), chitosan (CS) and water to form a hydrogel.

The biocompatible polymer mixture forming a hydrogel may comprise only chitosan (CS) and polyethyleneimine (PEI) as polymer components, present in the ratio of from 10:90 to 90:10.

The biocompatible polymer mixture forming a hydrogel may be formed in a cell culture medium.

According to a seventh aspect the present invention provides an implant for use in the repair or replacement of living tissue comprising a biocompatible polymer mixture according to the description of the sixth aspect given above.

The implant according may further comprise living cells attached to the biocompatible polymer mixture.

Alternatively the implant may further comprise tissue produced by incubation in vitro.

The implant may further comprise additional components selected from the group consisting of growth factors, DNA,RNA, proteins, peptides and therapeutic agents for treatment of disease conditions.

The implant may be of a biocompatible polymer mixture that is biodegradable and also further comprise a non-biodegradable component.

Such an implant may be a a stent of a non-biodegradable material that is coated with the biodegradable biocompatible polymer mixture.

According to an eighth aspect the present invention providesa method for repair or replacement of tissue comprising: providing an implant of the seventh aspect and locating the implant on or in the body of a subject. According to a ninth aspect the present invention provides a microarray comprising a plurality of polymer mixtures according to the sixth aspect.

The microarray may have agarose as the substrate.

The microarray may be made by a method comprising:
preparing a plurality of solutions comprising the polymer components; and
printing said solutions as an array of microspots onto a substrate; and
drying the microspots.

According to another aspect the invention provides a method of making a biocompatible polymer mixture according to the invention comprising:
preparing a solution of selected polymer components in a suitable solvent; and
removing the solvent from the mixture.

### Brief description of the Drawings

Figs. 1 (A to H) shows scanning electron micrographs of microarrays of polymer mixtures;
Figs. 2 (A to D) show visualisation of cells attached to a microarray of polymer mixtures;

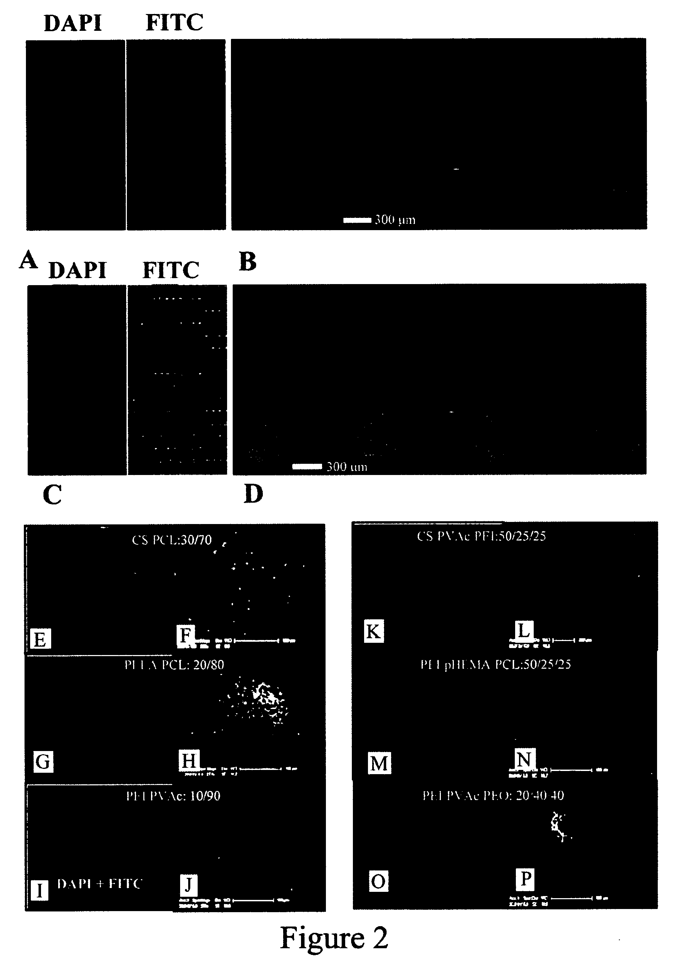

Figs. 2 (E to P) show visualisation of cells attached to microarray spots of various polymer mixtures and their corresponding SEM images;

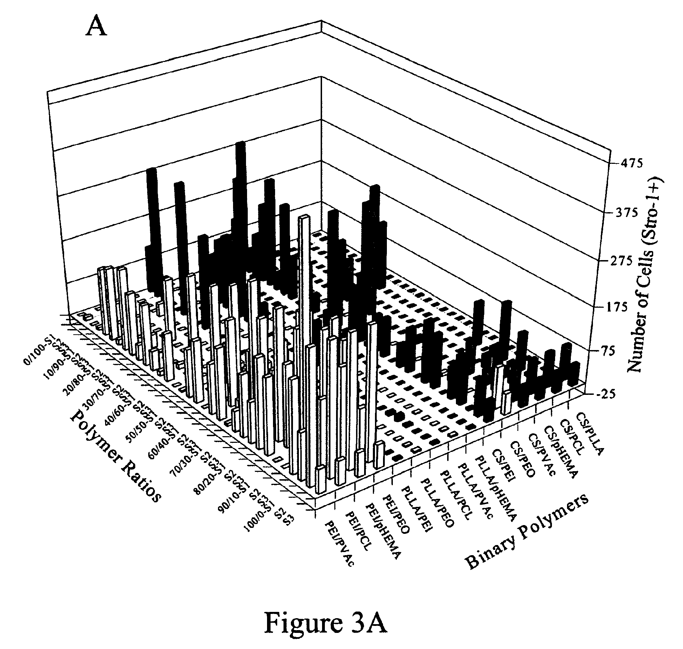

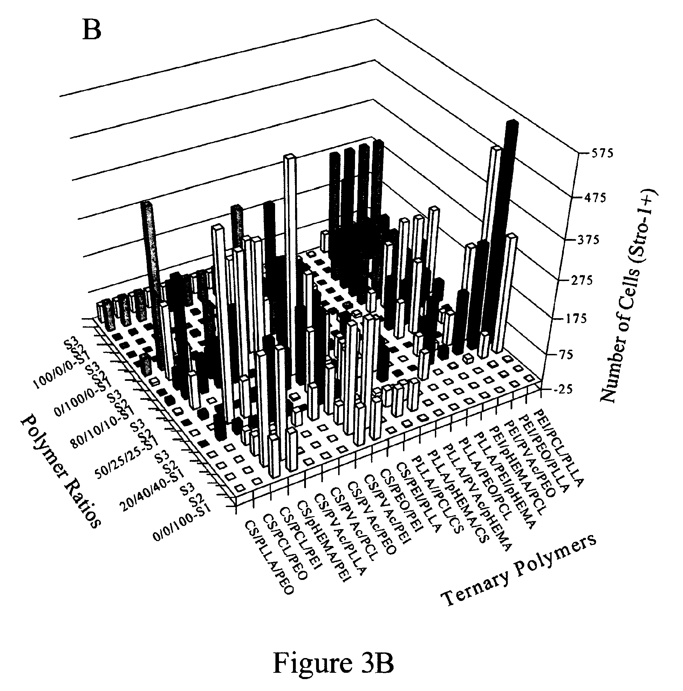

Figs. 3 (A and B) show graphically the extent of cell attachment for Stro-1+ cells found for various polymer mixtures of the invention;

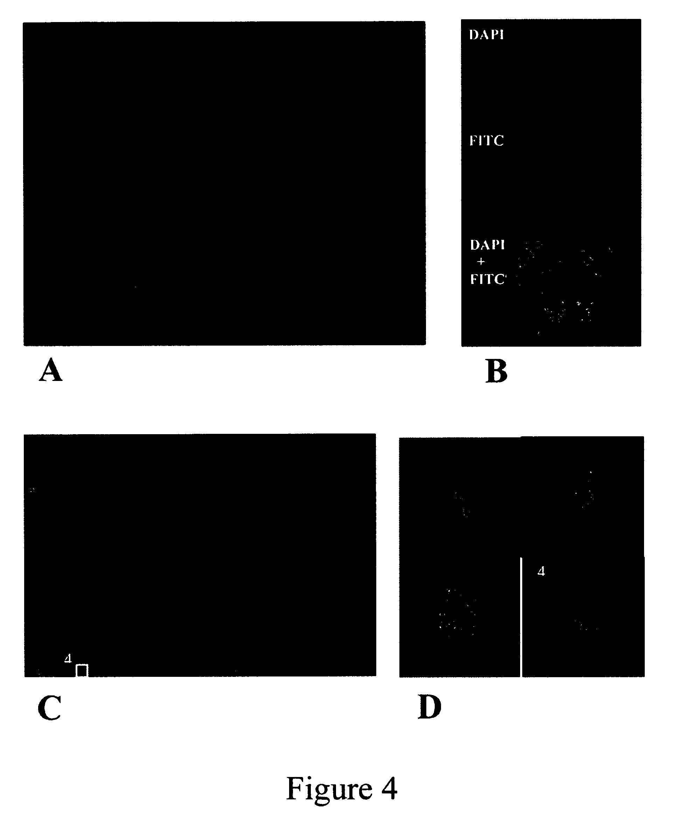

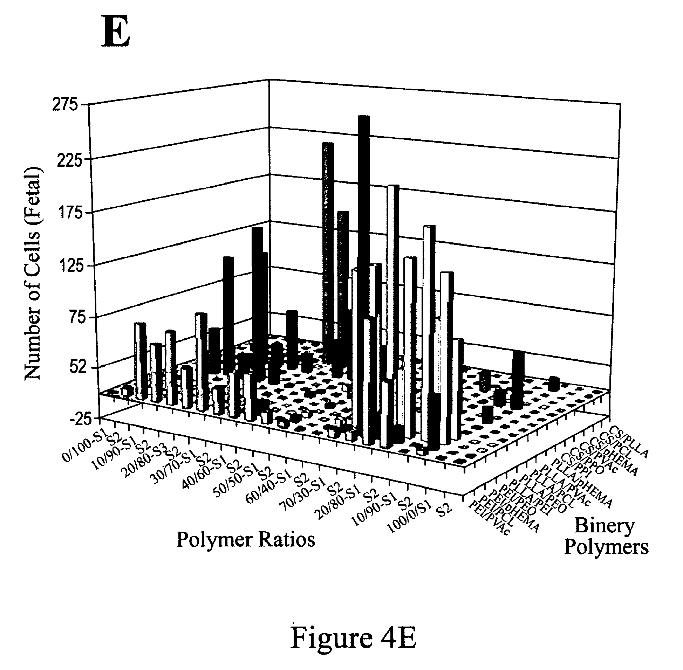

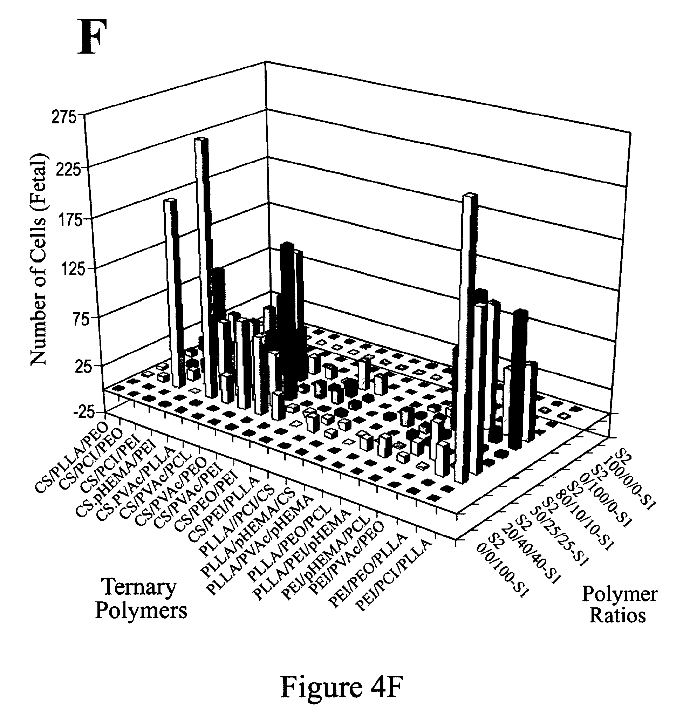

Figs. 4 (A to D) show visualisation of skeletal fetal cells attached to microarrays of polymer mixtures;
Figs. 4 (E and F) show graphically the extent of cell attachment for skeletal fetal cells found for various polymer mixtures of the invention;

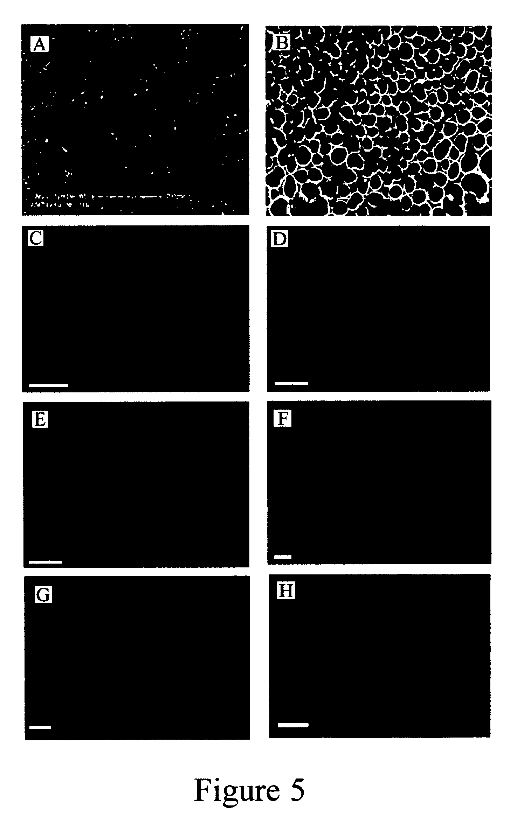

Figs. 5 (A and B) show detailed SEM images of polymer mixtures of the invention;
Figs. 5 (C to H) show images of skeletal cells (Stro-1+ labelled with cell tracker green) growing in polymer mixtures of the invention;

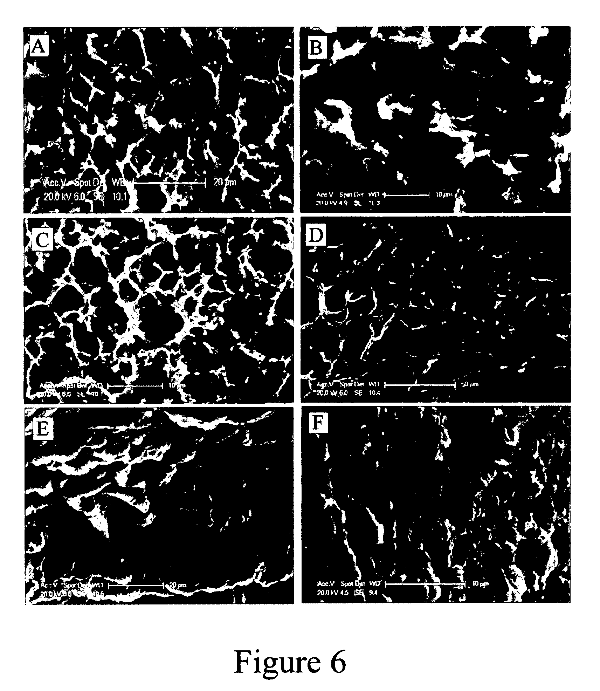

Figs. 6 (A to F) show SEM images of polymers containing chitosan;

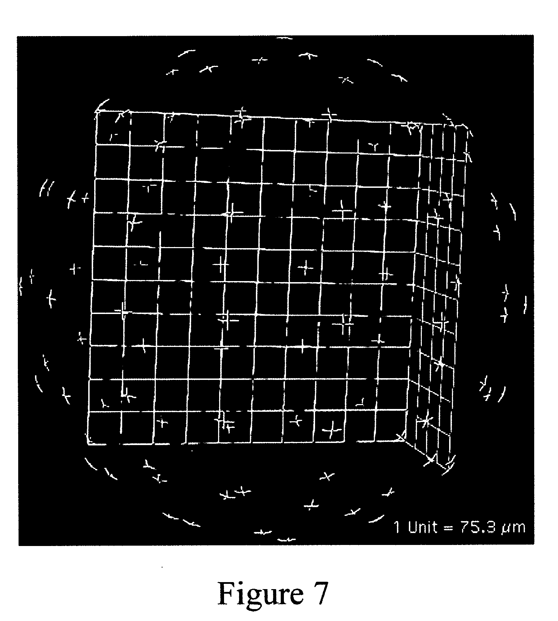

Fig. 7 shows HeLa cells labelled with Cell Tracker Green growing in a chitosan /polyethylenimine polymer (gel) mixture; and

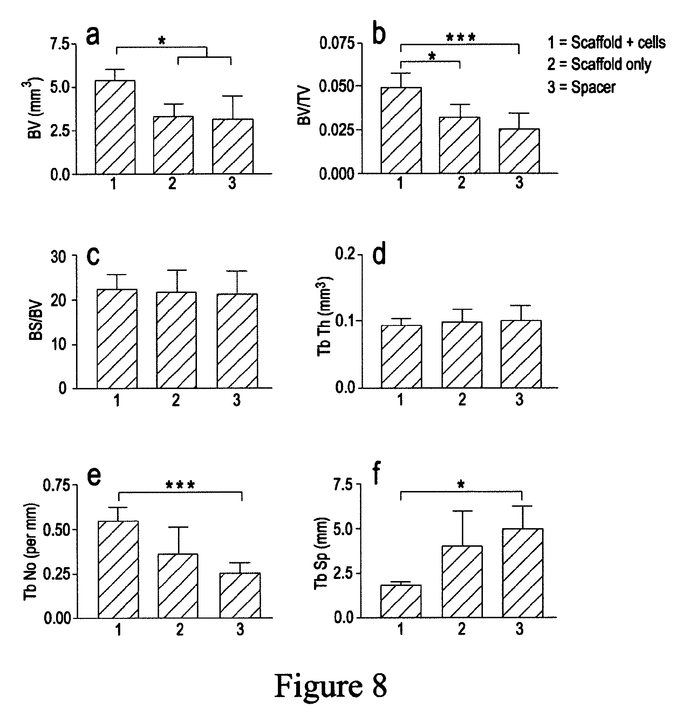

Fig.8 (a to f) shows graphically results from in vivo tests using polymer mixtures of the invention in bone repair.

### Detailed Description of the Invention with reference to the preparation of some exemplary compositions of the invention and results of tests

### MATERIALS AND METHODS FOR PREPARATION OF POLYMER BLENDS AND FABRICATION OF MICROARRAYS

**Materials:** Chitosan(CS) [75% deacetylated], polyethylenimine (PEI) and poly(N-isopropylacryalmide) (PNIPAM)and poly(lactide-co-glycolide)(PLGA) were purchased from Aldrich, poly(L-lactic acid) (PLLA), poly(D-lactic acid) (PDLA, poly[(R)-3- hydroxyl butyric acid] (PHB) from Fluka and the other polymers (PCL, PHEMA, PEO, CA, PVAc) from Sp² scientific polymer products, INC., and used without further purification. The solvents chloroform (CHCl₃), glacial acetic acid and N-methyl-2-pyrrolidinone (NMP) were analytical grade, purchased form Fisher scientific. 1, 4 Dioxane was purchased from across organics. Agarose (Type 1-B) and microscopic slides (Silane-Prep™) were obtained from Sigma-Aldrich.

**Polymer Solution Preparation:** Chitosan (1% w/v) was dissolved in 2% acetic acid solution in an aqueous medium, and this solution was then filtered to remove any foreign materials. The polyethylenimine and poly (2-hydroxyethyl methacrylate), poly(ethylene oxide)(PEO) and PNIPAM were dissolved in NMP. The other polymers (PCL, PLLA, PDLA, PHB, PLGA and PVAc) were dissolved in CHCl₃ or Dioxane and NMP with a ratio of 50/50 (v/v). All polymers were dissolved at a concentration of 1% in the solvents. These solutions were blended with different proportions and printed on agarose coated microscopic slides. The silane-prep™ slides were deep coated with agarose (1% w/v) solution and dried at ambient conditions for at least 24 hrs to obtain a stable film on the slide before being use for polymer microarray fabrication. The thickness of the agarose film was about 1.2 µm as measured by scanning electron microscopy.

**Polymer Microarray Fabrication:** The arrays of polymer mixtures were fabricated by contact printing (Qarray mini, Genetix) with 16 aQu solid pins (K2785; Genetix; UK) using 1.0 % polymer solutions (w/v) placed into polypropylene 384-wells micro plates. The following printing conditions were used, 5 stamping per spot, 200 ms inking time and 10 ms stamping time. The typical spot sizes was 300-400 µm in diameter with spot to spot distances of either 750 µm or 1120 µm (x-direction) and 900 µm or 2250 µm (y-direction) allowing up to 960 polymer spots to be printed on a standard 25 x 75 mm slide. The polymer mixtures were made into two arrays, one of which consisted of 142 mixtures comprising binary polymers with different proportions and the second array comprising ternary polymer mixtures with a total of 57, printed onto a conventional (25 x 75 mm) glass slide. These arrays can be used to immobilize a number of cell lines including fetal and human bone marrow stem cells onto individual polymer spots. Once printed, the slides were dried for 24 hrs under vacuum at 40 °C and sterilized under UV irradiation for 30 minutes prior to cell seeding.

**Scanning Electron Microscopy:** Scanning electron microscopy (Philips XL30CP SEM) was used to examine the surface morphology of the polymer spots. The glass slides containing the polymer arrays were covered with a thin layer of Au and glued to a metal-base specimen holder to achieve good electrical contact with the grounded electrode. The micrographs were taken at 10 kV in a secondary electron imaging mode.

**Differential Scanning Calorimeter:** Glass transitions (Tg) and the melting temperatures were determined using a TA Instruments DSC 2010 with both heat flow and temperature scales calibrated against indium metal. Nitrogen was used as purge gas and samples were scanned at 10 °C min⁻¹.

### CELL ISOLATION AND CULTURE

**Human bone marrow-derived STRO-1 immunoselected progenitor cells**: Bone marrow samples (n=3) were obtained from haematologically normal osteoarthritic and osteoporotic individuals undergoing routine hip replacement surgeries, with approval of the Southampton & South West Hampshire Local Research Ethics Committee. Skeletal progenitors from these bone marrow samples were immunoselected using the STRO-1 antibody, followed by isolation using MACS as described before¹⁹. The isolated STRO-1+ skeletal progenitors were expanded by culturing them to confluence in monolayers supplemented with 10% FCS in α-MEM.

**Human fetal skeletal progenitor cells:** Cartilaginous femora (n = 5) from human fetuses (age: 8-11 weeks post-conception) were obtained after the termination of pregnancies according to the guidelines issued by the Polkinghome Report and with ethical approval from Southampton & South West Hampshire Local Research Ethics Committee. Cells were isolated from these predominantly cartilaginous femora as described previously¹⁸ and cultured to confluence for 12 days in monolayers in basal medium (α-MEM containing 10% FCS).

**Human Osteosarcoma Cell Lines:** The two human osteosarcoma cell lines namely, MG63 and SaOs were cultured in monolayers in basal medium (10% FCS in DMEM and α-MEM respectively) up until cultures were -70% confluent. Cells harvested from these cultures were incubated on the polymer microarrays (n = 2 for each cell line).

**Protocol for studying cell - polymer interactions using polymer microarrays:** The polymer microarrays were sterilized under UV light for 30 min. STRO-1+ bone marrow-derived adult skeletal progenitor cells, human fetal skeletal progenitors and the two osteosarcoma cell lines were fluorescently labelled with Cell Tracker Green/ CMFDA. Suspensions of these cell populations in 1.5 ml basal media were pipetted onto the microarrays (10⁶ cells/ microarray slide), so that the entire microarray surface was covered with a thin film of cell suspension. The cells were incubated on the microarrays overnight at 37°C, 5% CO₂ in humidified atmosphere. The following day, microarrays were thoroughly washed using PBS to remove any unbound/ loosely-bound cells and the cells bound to the polyurethane spots were fixed using 4% formalin solution in PBS. Nuclei of cells bound to the polymer spots were stained using DAPI and the microarrays were stored in PBS at 4°C until analysis.

**Image Capture and High Throughput Analysis:** Images of arrays were captured with a Zeiss Axiovert 200 fluorescence microscope at x 20 magnifications and analyzed using Pathfinder™ software (IMSTAR S.A., Paris, France).

### Characterization of a Polymer Library

The phase transition behaviors of the polymers were determined by differential scanning calorimeter (DSC).

Most of the polymer mixtures showed at least two transition temperature phases with distinct values of individual components. As expected, a polymer containing crystalline components (e.g., PLLA, PCL and PEO), showed variation of crystallinity when blended with another polymer. In the case of amorphous polymers (PHEMA, CS, PEI and PVAc), the *Tg* of the polymer changes. These behaviors are due to the interactions between the components present in the mixture.

The driving force of the miscibility of the polymers is believed to be the hydrogen bonding, Van der Waal's, hydrophilic and hydrophobic interactions between the polymer chains. In comparison to the individual (pure) polymers morphological changes take place in the mixtures, resulting in the improved properties for cell attachment and growth.

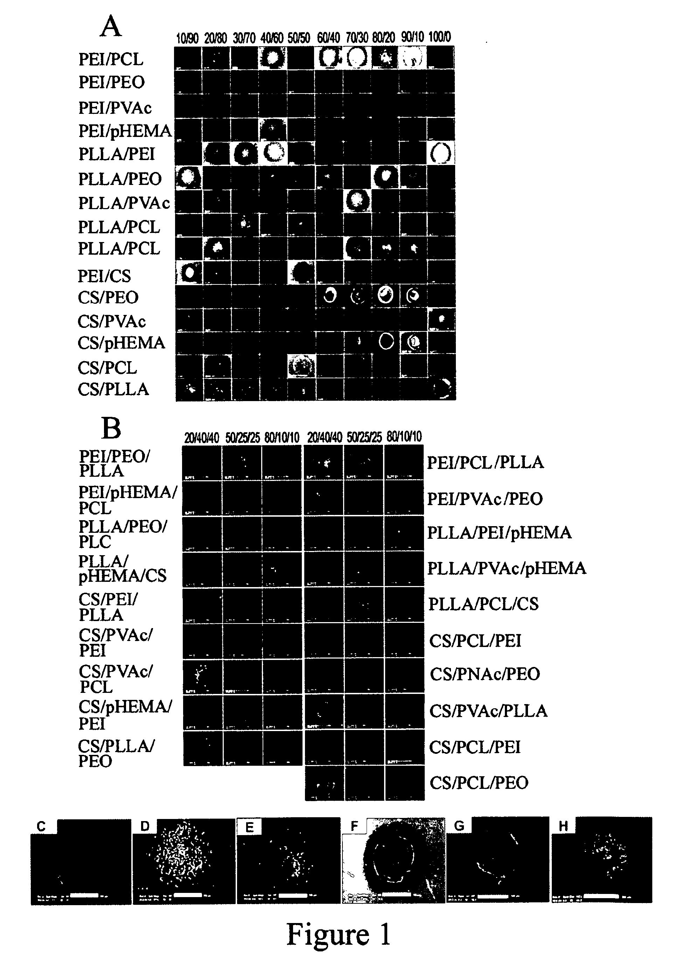

The morphology and phase behavior of the polymer mixtures were also examined by SEM. In a first set of experiments, compounds containing two-component polymers (a binary mixture) were studied. Figure 1A shows an SEM of an array of binary polymer mixtures. The blending of amorphous polymers, especially blending of PEI with PHEMA and PVAc shows a clear and transparent morphology. Blends of PEI with CS formed complex fibrous networks (see Fig 1F a 50:50 mixture). In some cases, the blending of a crystalline polymer with an amorphous polymer produced crystalline morphology in the mixture, depending on the compositions. Fig 1 C-F shows the following binary mixtures in closer detail:- (C) PLLA/PEO: 50/50 blend showing the phase separation. (D) PLLA/PHEMA (30/70) mixture showing crystalline phase separation of PLLA. (E) PLLA/PCL (50/50) blend. (F) CS/PEI (50/50) blend.

During the processing of these blends, it was noted the blending of CS with PEI for all compositions produced hydrogels. When dried an interpenetrated fibre formation was observed (Fig. 1 F).

In a second set of experiments, mixtures with three component polymers (ternary blends) were prepared and their SEM images are presented in an array format (Figure 1B) which show some differences in morphology as compared to those of binary mixtures. Figures 1G and H show the following mixtures in closer detail:- (G) CS/PEO/PEI: 20/40/40. (H) PEI/PCL/PLLA: 50/25/25.

The average size of the polymer spots was ∼500 µm, in the case of ternary polymer mixtures (the average spot sizes was ∼350 µm for binary mixtures). Spot size can be adjusted as desired by changing the printing conditions employed.

In the solution blending process employed, the solubility of each polymer in the solvents can be different, which can lead to different phases (regions containing different proportions of polymer component) within the polymer mixture produced.

The two-component polymer mixtures and three-component polymer mixtures were printed on separate chips to avoid merging spots.

The polymer microarray chips were examined for cell attachment and growth, by testing the arrays with osteioprogenitor cell lines such as fetal femur and mesenchymal stem (STRO-1⁺) cells.

STRO-1⁺ cells were immobilized on the polymer blends microarrays with results shown in Figure 2. Human skeletal stem cells (STRO-1⁺) were grown on the polymer blends arrays, with one million cells immobilized with Cell Tracker Green on the polymer microarray slide for 18 hours followed by washing with PBS. Cells were then fixed and nuclei stained with Hoechst-33342.

Figure 2 shows:-(A) STRO-1⁺ cells bound to the array of 142 binary polymer mixtures with six replicates. (B) Shows the STRO-1⁺ cells attached on the six replicates of the same polymer mixtures (highlighted box in A, PLLA/PCL: 20/80). (C) STRO-1⁺ cells bound to the array of 57 ternary polymer mixtures with four replicates. (D) shows quadruplicates of PEI/PCL/PLLA: 20/40/40.

Figures 2(E-P) illustrate the range of high to low binding affinities found for cells together with corresponding SEM images of the polymer spots. High binding affinity was found for (E) a CS/PCL (30/70) mixture and (K) CS/PVAc/PEI (50/25/25) with corresponding SEM images of the spots also shown (F, L). A medium binding affinity was found for (G) PLLA/PCL (20/80) and (M) PEI/PHEMA/PCL (50/25/25) with corresponding SEM images of the spots also shown (H, N). Low binding affinity was found for (I) PEI/PVAc (10/90) and (O) PEI/PVAc/PEO (20/40/40) with corresponding SEM images of the spots also shown(J,P).

Figure 2A shows the STRO-1⁺ attachment and spreading (growth) on the 142 binary polymer blends. With six replicates, it was calculated that 50% of the blends supported STRO-1⁺ cell attachments. Cell attachment on the ternary polymer blends was found to be more efficient (65% ternary blends showed STRO-1⁺ cells attachment) with respect to the binary blends (Fig 2C). Results (Figs 2B & 2D) indicated that the affinity of STRO-1⁺ binding on the polymer spots is highly reproducible and selective.

Cell compatibility was evaluated in terms of the total number of cells immobilised onto each polymer spot which was identified using the DAPI and FITC channels using Pathfinder™ software. The cell attachment and spreading on the replicates of identical polymer mixtures are very similar, examples are PLLA/PCL (20/80) (Fig. 2B, average number of cells 315) and PEI/PCL/PLLA (20/40/40) (Figure 2D, average number of cells 565).

The analysis of the number of Stro-1+ cells immobilized on each polymer spot was evaluated, and plotted (Fig.3A binary mixtures, Fig.3B ternary mixtures) as a function of compositional gradient of the polymer. It shows that the PEI/PVAc, PEI/PHEMA, PEI/PEO, PLLA/PCL and CS/PEI blends have an exceptionally high number of cells attached on the polymer spots. The identification of suitable materials that selectively support the attachment and growth of skeletal progenitors using these microarrays can be readily used to determine tissue engineering constructs by employing different polymer blends and cell-types.

Figure 4 shows the results of experiments, using skeletal fetal cells, carried out in similar fashion to those of figure 3. Figure 4 shows:-(A) fetal cells bound to the array of 142 binary polymer mixtures with six replicates. (B) Cells attached on the polymer spot CS/PCL:30/70 mixture (highlighted box in A) showing high-resolution pictures of cells nuclei stained with Hoechst-33342 (DAPI), FITC and their combinations. (C) Cells bound to the array of 57 ternary polymer mixtures with four replicates. (D) Four ternary polymers mixtures: CS/PVAc/PLLA: 20/40/40 (1), CS/PVAc/PCL: 80/10/10 (2), PEI/PVAc/PEO: 20/40/40 (3), PEI/PCL/PLLA: 80/10/10 (4) (as highlighted in 4C). (E) and (F) shows graphical analysis of the cell attachment as a function of binary and ternary polymer compositions.

For both binary and ternary polymer mixtures fetal cell attachment and growth was only observed on the polymer mixtures. The analysis of the cell attachment to the polymer spots was performed by calculating the number of cells and plotting them as a function of compositional gradients (Fig 4E binary mixtures, Fig 4F ternary mixtures). The results indicate that many of the ternary polymer mixtures have the same ability to support human fetal skeletal cells attachment as do binary mixtures.

To examine the effect of the polymer mixtures on the other cell types, microarrays of polymer blends of the invention were seeded with human osteosarcoma (SaOs) and osteoblast-like (MG63) and ATDC5 cell lines. The SaOs and MG63 cells attached to most of the mixtures, including PEI based mixtures, in the same way as the STRO-1⁺ cells. Unlike the other cell lines, the behaviour of the ATDC-5 differed significantly; these cells grew only on a few mixtures.

The high efficiency of cell attachment to certain mixtures can be due to a wide range of physical and chemical factors of the polymer surface. However this result emphasises the ability of the microarray approach to quickly allow evaluation of different cell lines against different polymer mixtures.

It is important to design cell-compatible biomaterials, which are resistant to aqueous based conditions i.e. which do not simply dissolve in an aqueous environment. PEI alone is soluble in water and also toxic for cells. Blending with other polymers such as PVAc, PEO, etc., resulted in mixtures with improved aqueous resistance and allowed compatibility with cells.

Figure 2 indicates that most of the mixtures prepared by mixing of PEI with other polymers gave significant cell attachment. The binding efficiency of these cells was noticeably lower in the case of CS-based mixtures as compared to those of PEI-based materials (Figure 3). Moreover, some mixtures prepared from PLLA and PCL showed significantly high levels cellular attachment, whereas the corresponding homopolymers alone (PLLA and PCL) did not support skeletal cell attachment.

From this investigation, preferred mixtures such as CS/PEI, PEI/PVAc, PEI/pHEMA, PEI/PEO and PLLA/PCL were selected for further investigations for tissue engineering applications.

### Further investigation of selected mixtures

Mixtures were prepared with high concentrations of polymers in suitable solvents, typical concentration was 10%, and coated on a glass surface using a spin coater, with different ratios of the polymers to investigate the morphological structure of these materials.

Materials of interest prepared from PLLA and PCL, chosen by their ability to support skeletal cell growth in the first screen (Figures 2-4), were tested for use in scaffold fabrication. Both these polymers were dissolved in a common solvent (CHCl₃) and blended at room temperature. The solution of the mixture was coated on a glass surface by spin coater and followed by drying at ambient conditions.

Figure 5 illustrates the results as follows:- Figures 5A and 5B show SEM images of a PLLA /PCL: 50/50 mixture with a porous morphology. Figures 5C and 5D show (by fluorescence microscopy) Stro-1+ cells growing within a PLLA/PCL: 20/80 scaffold on culture day 12 (C) and culture day 21 (D). Figures 5 E and 5F show growth within a PLLA/PCL: 50/50 scaffold on culture day 12 (E) and culture day 21 (F). Figures 5G and 5H show growth within a PLLA/PCL: 80/20 scaffold on culture day 12 (G) and culture day 21 (H).

A porous morphology was observed, as depicted by the SEM micrographs (Figure 5 A,B). Most of the pore sizes in the PLLA/PCL mixtures (20/80, 50/50, 80/20) were found to be in the range of between 10 µm and 25 µm, which is regarded as suitable for cell encapsulation, growth enhancement, proliferation and differentiation for tissue regeneration purposes.

After preparing the 3-D porous structure of polymer mixture, human skeletal cells were seeded for a culture period of up to three weeks. Cell viability and growth were monitored by fluorescence microscopy with the results as shown in figures 5C to H.

Further studies showed that 3D scaffolds of PLLA/PCL functioned as excellent biomimetic templates and promoted skeletal stem cell attachment and growth in long-term (21-day) in vitro cultures with excellent viability observed. In the absence of osteogenic induction factors, culture of STRO-1⁺ skeletal stem cells on these polymer blend scaffolds (for 21 days) were observed to undergo osteogenic differentiation, evidenced by the expression of alkaline phosphatase - an early marker of osteogenic differentiation. Moreover, further osteogenic differentiation of the STRO-1⁺ skeletal progenitors into mature osteoblasts, initiated by recombinant human bone morphogenic protein (rhBMP-2) ascorbate and dexamethasone, was supported by this polymer blend scaffold as illustrated by the expression of collagenous and non collagenous bone matrix proteins namely, Type I collagen, Bone Sialoprotein, Osteopontin, Osteonectin and Osteocalcin by cells from day 28 explants. Thus, under osteogenic conditions in *vitro,* polymer blend scaffolds composed of the PLLA/PCL (20/80) facilitated the generation of a mature osteoblast population from the STRO-1⁺ skeletal stem cell population indicating the suitability of the PLLA/PCL (20/80) scaffold as a temporal substrate supporting fundamental activities of skeletal stem cells including attachment, migration, proliferation and osteogenic differentiation.

Other mixtures (e.g. PEI blended with PEO, PHEMA and PVAc) were also further investigated. All these materials could be useful in controlling cellular architecture as well as in manipulating cellular function.

### In vivo studies

To investigate the bone regeneration potential of human bone marrow-derived STRO-1⁺ skeletal stem cells seeded onto 3D scaffolds of PLLA/PCL (20/80) in vivo, an innovative mouse critical-sized femur defect model (load-bearing) was employed²⁷. Two-dimensional *in vivo* digital X-rays and 3D detailed images of entire femora generated by µCT analysis were utilised for the initial evaluation of the extent and degree of bone healing in femoral defects, 28 days postoperatively. Defects were implanted with either scaffolds seeded with cells or scaffolds without cells or inert spacers as control.

Defect regions in femora implanted with scaffolds seeded with human skeletal stem cells demonstrated significant bone regeneration, confirmed by histology, which illustrated significant region cell infiltration to the defect implanted with the scaffold and extensive cell-generated unmineralized bone matrix/ osteoid. The defect regions in femora implanted with scaffolds alone exhibited some degree of bone regeneration, evidenced by histological analysis of the defect region, which demonstrated remnants of the PLLA/PCL blend scaffold and newly synthesized unmineralized bone matrix/ osteoid. In comparison to the femoral defects implanted with the cell-seeded scaffolds or scaffolds alone, negligible bone repair was seen in femoral defects implanted with rubber spacers.

High resolution µCT scans at day 28 clearly indicated the extent of new bone formation in the defect regions implanted with the PLLA/PCL blend scaffolds seeded with STRO-1⁺ skeletal stem cells, compared to the PLLA/PCL blend scaffold alone group and the control (rubber spacer) group. A significant increase in bone volume (BV) was observed in the defect regions implanted with cell-seeded scaffolds (5.399mm³ ± 0.625) compared to PLLA/PCL scaffold only (3.351mm³ ± 0.676, **P*< 0.05) and the control (spacer) groups (3.115mm³ ± 1.379, **P*< 0.05) (Fig. 8a). Similarly, the ratio of bone volume/ total volume (BV/ TV) showed a significant increase in the PLLA/PCL scaffolds seeded with STRO-1⁺ cells (0.049 ± 0.009) compared to the PLLA/PCL scaffold only group (0.032 ± 0.007, **P*< 0.05) and the control (spacer) group (0.025 ± 0.009, ****P*< 0.001) (Fig. 8b). Furthermore, examination of trabecular number (Tb No) showed an increase in the number of bone trabeculae in the defect regions associated with the cell-seeded PLLA/PCL scaffolds (0.540 ± 0.076) compared to the PLLA/PCL scaffold alone group (0.355 ± 0.155, P< 0.076) and a highly significant increase in comparison to the control (spacer) group (0.247 ± 0.061, ****P*< 0.001) (Fig. 8e). In support of the observed increase in BV, BV/ TV and Tb No, a decrease in trabecular spacing (Tb Sp corresponding to the distance between adjacent trabeculae) was observed in the femoral defect region implanted with the cell-seeded PLLA/PCL scaffolds (1.820 ± 0.204) compared to the PLLA/PCL scaffold alone group (4.004 ± 1.952, P< 0.0676) and the control (spacer) group (4.905 ± 1.328, **P*<0.05) (Fig. 8f). Examination of bone surface to bone volume (BS/BV) (Fig. 8c) and trabecular thickness (Tb Th) (Fig. 8d) showed no significant differences between the groups.

Histological analysis of defect regions in femora implanted with scaffolds seeded with human skeletal stem cells demonstrated substantial cell infiltration in the region of the defect and extensive cell-generated unmineralized bone matrix/ osteoid rich in Type I collagen. The defect regions in femora implanted with scaffolds alone also exhibited some degree of bone regeneration, evidenced by histological analysis of the defect region, which demonstrated layers of cells surrounding remnants of the PLLA/ PCL scaffold and newly synthesized unmineralized bone matrix/ osteoid immunostained for the presence of Type I collagen. In comparison to the femoral defects implanted with the cell-seeded scaffolds or scaffolds alone, histologically, negligible bone repair was seen in femoral defects implanted with rubber spacers.

### Test results for polyethylenimine chitosan mixtures

Polymer mixtures were generated by mixing chitosan (partially hydrolyzed, Mw 250 kDa, 1% aqueous acetic acid, pH - 4.0) and polyethylenimine (Mw 300 kDa, 10% in water, pH -11) in various molar ratios (90/10 to 10/90). The resulting solutions (pH -7.5) became, over a period of 5 minutes, gels stable to inversion and manipulation. All compositions showed gelation, however these varied from less opaque (chitosan/PEI: 10/90) to more opaque gels (chitosan/PEI: 40/60).

The resulting hydrogels were examined by a range of physical techniques, including scanning electron microscopy (SEM), IR, Rheometer and XRD. The hydrogel prepared from chitosan/PEI (40/60) displayed a sponge-like, microporous morphology, radically different to that found in "normal" chitosan gels (chitosan homopolymer) prepared by neutralization of solubilised chitosan and suggested possible application as a cellular support/scaffold.

SEM images (Figure 6 A to D) demonstrate the porous structure of the mixtures prepared by using chitosan and PEI. The blending of PEI with chitosan can form hydrogels, in an aqueous phase.

It was also noted that both small pores and large pores were produced. The number of large pores and the sizes of the pores increase with increasing CS to PEI ratio. These results show that changing the proportion and concentrations of PEI in chitosan can be used control the porous morphology.

The SEM image shown in Figure 6(E) illustrates the morphology of a chitosan/PEI mixture having a ratio of 10/90. Figures 6(A & B) and 6(C & D) show images of preferred chitosan/PEI mixtures(30/70 and 40/60 ratios respectively). Figure 6(F) shows a chitosan only gel (chitosan solution neutralized with NaOH).

Gel samples were also characterized by XRD which indicated that a gel prepared by NaOH neutralization of chitosan, gave a crystalline material, which was radically different to the gel based materials made by blending the two cationic polymers (PEI and chitosan). FTIR analysis of the gels also indicated major changes following gelation, indicating strong interactions between two components. This strong interaction on forming the hydrogels was also confirmed by mechanical testing which showed that the 40:60 chitosan/PEI hydrogel had a high compressive modulus and a high storage modulus (relative to the individual polymers) when formed.

Microarray testing of chitosan polymer mixtures as described above showed that most mixtures tested showed significant cell attachment, in contrast to the homopolymers.

The preferred mixture (CS/PEI: 40/60) showed very good cell encapsulation and proliferation which may be attributed to the open network structure shown in figures 6(C, D).

To investigate this utility further HeLa and primary human fetal skeletal cells²⁰ labelled with Cell-Tracker Green, were seeded and cultured within chitosan/PEI hydrogels over a period of 28 days.

The cell culture procedure was as follows:

Human skeletal cells were harvested from day 12 monolayer cultures grown in α-MEM supplemented with 10% FCS and labeled with Cell Tracker Green/ CMFDA (Invitrogen) following the manufacturer's instructions. 100,000 labelled cells were suspended in the sterile PEI solution and this solution was added immediately to the sterile chitosan solution, previously added to wells of a 24-well plate. Gelation of the mixture of the two solutions (chitosan/PEI: 40/60) took place within two minutes encapsulating the cells within the gel. Once this was achieved, 1 ml of media (α-MEM containing 10% FCS) was added to each well containing the gel, and the cells were cultured for a period of 28 days in this culture system. The media was replaced every two days, and cell viability and growth monitored on days 3, 7, 15, 21 and 28 of culture by fluorescence microscopy. For HeLa cell culture, RPMI-CM media (supplemented with 10% fetal bovine serum (FBS), glutamine (4 mM), and antibiotics (penicillin and streptomycin, 100 units/ml)) was used. The media was changed every two days.

The cells were found to be homogeneously distributed throughout the hydrogel scaffold (Figure 7 shows HeLa cells in a confocal image) and were viable, healthy and proliferated throughout the four-week culture period. It was noticed that the gels started to disintegrate when cell culture was prolonged beyond this.

Throughout the course of the 28-day culture period within the chitosan/PEI hydrogels, the human fetal skeletal cells, derived from predominantly cartilaginous fetal femora ²⁰ exhibited a chondrocyte-like spherical morphology, as opposed to the fibroblastic morphology found in monolayer culture. Expression of *Pcna,* a marker of cell proliferation,²⁶ by fetal skeletal cells was analysed (RT qPCR) to determine the effect of culture within chitosan/PEI hydrogels on cell proliferation.

The culture, in the absence of chondrogenic differentiation media, resulted in a significant increase in *Pcna* expression on days 14 (P<0.01), 21 (*P*<0.001) and 28 (*P*<0.05) of culture in comparison to day 7 of culture, thus skeletal cells proliferated actively over the 28-day culture period within chitosan/PEI hydrogels.

To investigate the influence of the three dimensional (3D) chitosan/PEI hydrogel system on the culture of fetal skeletal cells compared to a standard mono-layer culture, analysis of key genes involved in cellular proliferation 25 and differentiation was carried out²⁵ . The 3D gel facilitated cellular proliferation yet prevented de-differentiation of these cells into fibroblasts as demonstrated by their spherical morphology and low levels of *Col1a1* expression (a gene prolifically expressed by fibroblasts). In addition, there was a steady increase in the expression of two fibroblastic markers (*Col2a1* and *Aggrecan* was observed. In contrast, expression of *Col1a1* (*Type I collagen*) by fetal skeletal cells cultured in monolayers, increased steadily with time with negligible expression of the two chondrogenic genes (*Col2a1* (*Type II collagen*) and *Aggrecan* indicative of de-differentiation of the fetal skeletal cells under conditions of monolayer culture.

The culture within a 3D gel environment as opposed to a 2D monolayer culture environment, thus prevented de-differentiation of the fetal skeletal cells into fibroblasts by maintaining these cells in a chondrocyte-like spherical morphology. Furthermore, the 3D chitosan/PEI hydrogel culture system in combination with the chondrogenic growth factor TGF-β3, was essential to stimulate the differentiation of the fetal skeletal cell population along the chondrogenic lineage.

### References

1. Shi D. Biomaterials and Tissue Enginering: Bioactive Materials and Processing and Biocomposite Materials for Biotechnology (Springer, 2004).
2. Ratner, B. D., Hoffman, A. S., Schoen, F. J. & J. E. Lemons, Biomaterials Science, (Elsevier, San Diego, 2nd Ed 2004).
3. Langer, R. Where a pill won't reach. Scientific American 288, 50-57 (2003).
4. Morice, M. C. et al. A randomized comparison of a sirolimus-eluting stent with a standard stent for coronary revuscularization. N. Engl. J. Med. 346, 1773-1780 (2002).
5. Shirazi, M. et al. Design and in vitro evaluation of polymer-coated drug-eluting intracoronary stents, 30th Annual Meeting & Exposition of the Controlled Release Society, Glasgow, UK, 2003, p. 476.
6. Bernkop-Schnűrch, A., Hornof, M. & Guggi, D. Thiolated chitosan. Eur. J. Pharm. Biopharm. 57, 9-17 (2004).
7. Vacanti, J. P. & Langer, R. Tissue engineering: the design and fabrication of living replacement devices for surgical reconstruction and transplantation. Lancet 354, 32-34 (1999).
8. Ng, K. W., Khor, H. L. & Hutmacher, D. W. In vitro characterization of natural and synthertic dermal matrices cultured with human dermal fibroblasts. Biomaterials 25, 2807-2818 (2004).
9. Suh, J.-K. F. & Matthew, H. W. T. Application of chitosan-based polysaccharide biomaterials in cartilage tissue engineering: a review. Biomaterials 21, 2589-2598 (2000)
10. Qin, C. et al. Water-solubility of chitosan and its antimicrobial activity. Carbohydrate Polymers 63, 367 (2006)
11. Hubbell, J. A. Biomaterials in Tissue Engineering. NatureBiotechnol. 13, 565-576 (1995).
12. Karabanova, L.V. et al. Polyurethane/poly(hydroxyethyl methacrylate) semi-interpenetrating polymer networks for biomedical applications. J. Mater. Sci.: Mater. Med. 17, 1283-1296 (2006).
13. Simon Jr., C. G. et al. Combinatorial screening of cell proliferation on poly(L-lactic acid)/poly(D,L-lactic acid) blends. Biomaterials 26, 6906-6915(2005).
14. Yang X. B. et al. Evaluation of human bone marrow stromal cell growth on biodegradable polymer/bioglass composites. Biochem. Biophys. Res. Commun. 342, 1098-1107 (2006).
15. Dalby, M. J., Riehle, M.O., Johnstone, H., Affrossman, S. & Curtis, A. S. G. In vitro reaction of endothelial cells to polymer demixed nanotopography. Biomaterials 23, 2945-2954 (2002).
16. Hoogenboom, R., Meier, M. A. R. & Schubert, U. S. Combinatorial methods, automated synthesis and high-throughput screening in polymer research: past and present. Macromol. Rapid Commun. 24, 15-32 (2003).
17. Smith, J. R., Seyda, A., Weber, N., Knight, D., Abramson, S. & Kohn, J. Integration of combinatorial synthesis, rapid screening, and computational modeling in biomaterials development. Macromol. Rapid Commun. 25, 127-140 (2004).
18. Akinc, A., Lynn, D. M., Anderson, D. G. & Langer, R. Parallel synthesis and biophysical characterization of a degradable polymer library for gene delivery. J. Am. Chem. Soc.125, 5316-5323 (2003).
19. Yang, X. et al. Induction of human osteoprogenitor chemotaxis, proliferation, differentiation, and bone formation by osteoblast stimulating factor-1/pleiotrophin: Osteoconductive biomimetic scaffolds for tissue engineering. J. Bone Miner. Res. 18, 47-57 (2003).
20. Mirmalek-Sani, S.-H. et al. Characterization and multipotentiality of human fetal femur-derived cells-implications for skeletal tissue regeneration. Stem Cells 24, 1042-1053 (2006).
21. Dalby, M. J. et al. The control of human mesenchymal cell differentiation using nanoscale symmetry and disorder. Nature Mater. 6, 997-103 (2007).
22. Richardson, T. P., Peters, M. C., Ennett, A. B. & Mooney, D. J. Polymeric system for dual growth factor delivery. Nature Biotechnol. 19, 1029-1034 (2001).
23. (a) Ganji, F.; Abdekhodaie M. J.; A.-Ramazani S.A. J Sol-Gel Sci Techn. 2007, 42, 47-53. (b) Suh, F. J.-K.; Matthew, H. W.T. Biomaterials 2000, 21, 2589-2598.
24. Phillips, H.J.; Terryberry, J. E. Exp. Cell Res. 1957, 13, 341-347.
25. P. D. Benya, J. D. Shaffer, Cell 1982, 30, 215-224.
26. D. Jaskulski, J. K. deRiel, W. E. Mercer, B. Calabretta, R. Baserga, Science 1988, 240, 1544-1546.
27. Kanczler, J.M. et al. The effect of mesenchymal populations and vascular endothelial growth factor delivered from biodegradable polymer scaffolds on bone formation. Biomaterials 29, 1892-1900 (2008).

## Claims

1. A biocompatible polymer mixture comprising a mixture of at least two polymers selected from the group consisting of:
poly (L-lactic acid) (PLLA), poly (ε-caprolactone) (PCL) , poly(D-lactic acid) (PDLA) , poly(2-hydroxyethyl methacrylate) (PHEMA), poly (vinyl acetate) (PVAc), poly [(R)-3-hydroxybutyric acid)] (PHB), poly(lactide-co-glycolide) (PLGA) , poly(N-isopropylacrylamide) (PNIPAM), cellulose acetate (CA), poly(ethylene oxide) (PEO), polyethylenimine (PEI), and chitosan (CS);
for use in the repair or replacement of bone or cartilage.

2. The biocompatible polymer mixture for use according to claim 1 wherein the mixture comprises two polymer components, selected from the group consisting of poly(L-lactic acid)/poly(ε-caprolactone) (PLLA/PCL), polyethylenimine/ poly(vinyl acetate)(PEI/PVAc), polyethylenimine/poly(2-hydroxyethyl methacrylate) (PEI/PHEMA), polyethylenimine/poly(ethylene oxide) (PEI/PEO),polyethylenimine /chitosan (PEI/CS), polyethyleneimine/ poly(ε-caprolactone)(PEI/PCL), poly(D-Lactic acid)/chitosan (PDLA/CS), poly(D-Lactic acid)/polyethylenimine (PDLA/PEI) poly[(R)-3-hydroxybutyric acid)]/chitosan (PHB/CS), poly[(R)-3-hydroxybutyric acid)]/ polyethylenimine (PHB/PEI), cellulose acetate / polyethylenimine (CA/PEI), cellulose acetate/chitosan (CA/CS), poly(Lactide-co-glycolide)/ chitosan (PLGA/CS), poly(Lactide-co-glycolide)/polyethylenimine (PLGA/PEI), poly(N-isopropylacrylamide)/chitosan(PNIPAM/CS), poly(N-isopropylacrylamide)/ polyethylenimine (PNIPAM/PEI), poly(N-isopropylacrylamide)/ poly(ε-caprolactone) (PNIPAM/PCL) , poly(N-isopropylacrylamide)/ poly(L-lactic acid)(PNIPAM/PLLA), poly(N-isopropylacrylamide)/ poly(D-Lactic acid)(PNIPAM/PDLA), poly(N-isopropylacrylamide)/ poly[(R)-3-hydroxybutyric acid)](PNIPAM/PHB), poly(N-isopropylacrylamide)/cellulose acetate(PNIPAM/CA), poly(N-isopropylacrylamide)/ poly(2-hydroxyethyl methacrylate)(PNIPAM/PHEMA), and poly(N-isopropylacrylamide)/ poly(vinyl acetate)(PNIPAM/PVAc).

3. The biocompatible polymer mixture for use according to claim 2 wherein the two polymer components are selected from the group consisting of: poly(L-lactic acid)/poly(ε-caprolactone) (PLLA/PCL), polyethylenimine/ poly(vinyl acetate)(PEI/PVAc), polyethylenimine/poly(2-hydroxyethyl methacrylate) (PEI/PHEMA), polyethylenimine/poly(ethylene oxide) (PEI/PEO),polyethylenimine /chitosan (PEI/CS), polyethyleneimine/ poly(ε-caprolactone)(PEI/PCL).

4. The biocompatible polymer mixture for use according to claim 2 wherein the mixture has a microporous structure and comprises two polymer components: poly(L-lactic acid) (PLLA) and poly(ε-caprolactone) (PCL); in particular wherein the polymer components of the mixture are poly(L-lactic acid)/poly(ε-caprolactone) (PLLA/PCL) having a ratio in the range from 10:90 to 60:40.

5. The biocompatible polymer mixture for use according to claim 4 wherein the polymer components of the mixture are poly(L-lactic acid)/poly(ε-caprolactone) and the poly(L-lactic acid)/poly(ε-caprolactone) (PLLA/PCL) is in the ratio of 20:80.

6. The biocompatible polymer mixture for use according to claim 1 wherein the mixture has a microporous structure and comprises two polymer components:
polyethylenimine (PEI) and chitosan (CS).

7. The biocompatible polymer mixture for use according to claim 1 wherein the biocompatible polymer mixture is a two component mixture containing polyethylenimine (PEI) and having a ratio in the range from 10:90 to 90:10 of PEI to the second component.

8. The biocompatible polymer mixture for use according to claim 1 wherein the mixture comprises three polymer components selected from the group consisting of PEI/PCL/PLLA, PEI/PEO/PLLA, PEI/PVAc/PEO, PEI/pHEMA/PCL, PLLA/PEI/pHEMA, PLLA/PEO/PCL, PLLA/PVAc/pHEMA, PLLA/pHEMA/CS, PLLA/PCL/CS, CS/PEI/PLLA,CS/PVAc/PLLA, CS/PEO/PEI, CS/PVAc/PEI, CS/PVAc/PEO, CS/PVAc/PCL, CS/pHEMA/PEI, CS/PCL/PEI and CS/PLLA/PEO.

9. The biocompatible polymer mixture for use according to claim 1 wherein the biocompatible polymer mixture comprises polyethylenimine (PEI), chitosan (CS) and water to form a hydrogel; and optionally,
wherein the hydrogel is formed in a cell culture medium.

10. The biocompatible polymer mixture for use according to claim 9 wherein the mixture comprises only chitosan (CS) and polyethyleneimine (PEI) as polymer components and they are present in the ratio of from 10:90 to 90:10.

11. A bone or cartilage implant, for use in the repair or replacement of bone or cartilage, comprising a biocompatible polymer mixture as described in anyone of claims 1 to 10.

12. The implant according to claim 11 further comprising living cells attached to the biocompatible polymer mixture, or tissue produced by incubation in vitro.

13. The implant according to claim 11 or to claim 12 further comprising additional components selected from the group consisting of growth factors, DNA,RNA, proteins, peptides and therapeutic agents for treatment of disease conditions.

14. The implant according to any one of claims 11 to 13 wherein the biocompatible polymer mixture is biodegradable and the implant further comprises a non-biodegradable component.

15. The implant according to any one of claims 11 to 14 wherein the implant is a stent of a non-biodegradable material that is coated with the biodegradable biocompatible polymer mixture.
